# DEMANDE DE BREVET EUROPEEN

(11) **EP 2 556 824 A1**
(43) Date de publication de la demande: **13.02.2013**
(21) Numéro de dépôt: 12179774.0
(22) Date de dépôt: 09.08.2012
(51) Int. Cl.: A61K 9/00, A61K 9/20, A61K 31/165

(54) **Composition pharmaceutique solide pour administration buccale d'agomelatine**

(30) Priorité: 10.08.2011 FR 1102500
(71) Demandeur: Les Laboratoires Servier, 92284 Suresnes Cedex (FR)
(72) Inventeur: Tharrault, François, 45000 ORLEANS (FR); Poirier, Cécile, 45000 ORLEANS (FR); Fonknechten, Gilles, 45240 LIGNY LE RIBAULT (FR); Pean, Jean-Manuel, 45000 ORLEANS (FR)

(57) **Abrégé**

L'invention a pour objet une composition pharmaceutique buccale solide contenant de l'agomélatine destinée à une action systémique.

## Description

La présente invention a pour objet une nouvelle forme pharmaceutique solide pour l'administration par voie buccale d'agomélatine.

L'agomélatine ou *N*-[2-(7-méthoxy-1-naphtyl)éthyl]acétamide de formule (I) : 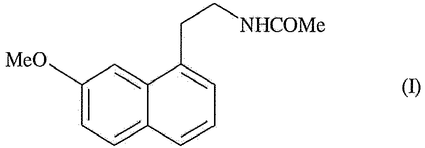 ainsi que ses hydrates, formes cristallines, complexes, co-cristaux, et sels d'addition à un acide ou à une base pharmaceutiquement acceptable, présente des propriétés pharmacologiques intéressantes: c'est un agoniste sélectif des récepteurs du système mélatoninergique et d'autre part un antagoniste du récepteur 5-HT_{2C}. Ces propriétés lui confèrent une activité dans le système nerveux central et plus particulièrement dans le traitement de la dépression majeure, des dépressions saisonnières, du trouble anxiété généralisée, du trouble obsessionnel compulsif, des troubles bipolaires, des troubles du sommeil, des pathologies cardiovasculaires, des pathologies du système digestif, des insomnies et fatigues dues aux décalages horaires, des troubles de l'appétit et de l'obésité.

L'agomélatine, ses formes cristallines, ses complexes, ses co-cristaux, ses sels d'addition à un acide ou à une base pharmaceutiquement acceptable, sa préparation et son utilisation en thérapeutique ont été décrits, entre autres, dans les demandes EP0447285, WO2005/077887, WO2007/015003, WO2007/015002, WO2007/015004, WO2010/097052, WO2010/102554, CN101955440, W02011/050742, CN102050756, WO2011/006387, WO2011/075943, CN101774937, CN101870662, CN102030673, WO2012/046253, WO2011/113362, WO2011/113363, CN102206864, CN102503886, et CN102432490.

Dans tout ce qui suit, par « agomélatine » on entend l'agomélatine ainsi que ses hydrates, ses complexes, ses co-cristaux, ses formes cristallines et ses sels d'addition à un acide ou à une base pharmaceutiquement acceptable.

L'agomélatine pourra en particulier être sous la forme cristalline II.

L'agomélatine peut être administré par voie orale ou plus précisément entérale sous forme de comprimés à libération immédiate à avaler avec un demi-verre d'eau. Ces comprimés d'agomélatine sont utiles, notamment pour le traitement de la dépression majeure, des dépressions saisonnières, du trouble anxiété généralisée, du trouble obsessionnel compulsif, des troubles bipolaires, des troubles du sommeil, des pathologies cardiovasculaires, des pathologies du système digestif, des insomnies et fatigues dues aux décalages horaires, des troubles de l'appétit et de l'obésité, et de l'ensemble des pathologies liées à un dérèglement des rythmes circadiens.

Des études pharmacocinétiques chez l'homme ont montré que la biodisponibilité de l'agomélatine par voie orale est faible par rapport à la voie parentérale et varie pour un même individu et d'un individu à l'autre.

Aussi, la biodisponibilité faible de l'agomélatine ainsi que les variations des concentrations inter et intra-individuelles ont conduit à rechercher une nouvelle formulation permettant de remédier à ces inconvénients. Une composition pharmaceutique solide orodispersible d'agomélatine décrite dans la demande de brevet EP1427724 a ainsi été mise au point contenant de l'agomélatine et des granules consistant en lactose et amidon séchés par co-atomisation et commercialisés sous l'appellation STARLAC^{®}. Cette composition pharmaceutique permet d'obtenir des comprimés ayant une très bonne aptitude à se désagréger dans la cavité orale et plus spécifiquement dans la cavité sublinguale, en respectant les critères d'orodispersibilité. Les comprimés orodispersibles permettent de délivrer le principe actif dans la cavité orale, et plus spécifiquement dans la cavité sublinguale, en moins de trois minutes. La dissolution du principe actif dans la salive puis l'absorption par les muqueuses de la cavité orale, et plus spécifiquement par la muqueuse sublinguale, et le passage rapide dans le sang permettent de contourner la dégradation présystémique et l'effet de premier passage hépatique. Ainsi, la biodisponibilité est très nettement améliorée avec des variabilités beaucoup plus faibles et une apparition rapide du principe actif dans le sang.
Cependant, il est rapidement apparu que cette formulation présentait un inconvénient provenant du principe actif utilisé, l'agomélatine, qui provoque une sensation irritante prononcée au niveau des muqueuses de la cavité orale.
Lorsque la voie sublinguale est spécifiquement ciblée, cet effet piquant est exacerbé en raison de concentrations locales élevées en agomélatine, ce qui se traduit par une très mauvaise acceptabilité par le patient. Dans ce contexte, une formulation de type comprimé sublingual a été mise au point et décrite dans la demande de brevet WO2007/068829, répondant aux exigences d'orodispersibilité et d'acceptabilité par le patient. La stratégie privilégiée a été l'obtention d'une composition pharmaceutique solide constituée d'un noyau central ou d'une couche centrale contenant de l'agomélatine et des excipients permettant d'obtenir une formulation orodispersible, et d'un enrobage orodispersible contenant éventuellement un contre irritant. Ces comprimés orodispersibles permettent d'obtenir une désagrégation rapide inférieure à trois minutes, et ont montré une excellente capacité à limiter le caractère piquant du principe actif.

La demanderesse a présentement suivi une nouvelle stratégie consistant à élaborer une formulation pharmaceutique permettant une libération progressive de l'agomélatine dans la cavité buccale. Le point important de cette nouvelle formulation est de maintenir tout au long de la libération du principe actif des concentrations locales suffisamment faibles pour limiter la sensation de piquant et obtenir une forme acceptable par le patient.
Ainsi une nouvelle formulation pharmaceutique a été mise au point permettant de pallier le problème irritant de l'agomélatine tout en privilégiant une délivrance du principe actif dans la cavité buccale en vue d'une action systémique, effet recherché pour améliorer les problèmes de biodisponibilité et de variabilité intra et inter-individuelle rencontrés avec les comprimés administrés par voie orale. De manière étonnante et totalement inattendue, cette nouvelle formulation a conduit à une augmentation de biodisponibilité du principe actif non seulement par rapport au comprimé oral (entéral), mais également par rapport aux formulations orodispersibles oromucosales développées dans l'art antérieur en vue d'une action systémique, pour des formulations de même dosage en principe actif et contenant le principe actif sous une même forme. Par « même forme » on entend, ici et à chaque fois que l'expression sera utilisée ci-après, que le principe actif a la même forme cristalline, ou est sous la même forme hydratée, complexée, salifiée ou co-cristallisée, et que les tailles des particules de principe actif sont les mêmes.

La présente invention concerne donc une préparation buccale solide destinée à une action systémique permettant une libération contrôlée du principe actif dans la cavité orale de façon à obtenir une bonne acceptabilité gustative et une meilleure biodisponibilité absolue par rapport aux comprimés orodispersibles à absorption oromucosale décrits dans l'art antérieur, pour des formulations de même dosage en principe actif et contenant le principe actif sous une même forme.
La préparation buccale selon l'invention concerne plus particulièrement les pastilles, les pâtes à sucer, les comprimés à sucer, les comprimés sublinguaux, les comprimés gingivaux, les solides obtenus par extrusion à chaud ou injection-moulage ou encore les sphères médicamenteuses destinées à se dissoudre dans la cavité orale, contenant de l'agomélatine.

La préparation buccale selon l'invention peut contenir des diluants, des liants, des agents d'écoulement, des lubrifiants, des agents d'enrobages, des plastifiants, des aromatisants, des contre-irritants et des édulcorants.

La préparation buccale selon l'invention est caractérisée par l'absence d'agent désintégrant qui, combiné à une force de compression élevée permet la conception d'un comprimé qui ne se désagrège pas, mais s'érode lentement par dissolution des composants à la surface.

La préparation buccale à action systémique selon l'invention présente un temps de libération du principe actif inférieur à 30 minutes, et plus préférentiellement inférieur à 15 minutes. La libération du principe actif se fera par exemple entre 3 minutes et 30 minutes, et plus préférentiellement entre 3 minutes et 15 minutes.

Les pastilles et les pâtes à sucer sont des préparations unidoses solides destinées à être sucées et à se dissoudre ou se désagréger lentement dans la bouche. Les pastilles à sucer sont des préparations dures obtenues par moulage. Elles contiennent le principe actif habituellement dans un excipient aromatisé et sucré. Optionnellement elles peuvent contenir des contre-irritants. Les pâtes à sucer sont des préparations molles et malléables obtenues par moulage de mélanges contenant des gommes ou polymères naturels ou synthétiques et des édulcorants. Optionnellement elles peuvent contenir des aromatisants et des contre-irritants.
Les pastilles et pâtes à sucer selon l'invention comprendront par exemple de l'agomélatine; de la gomme arabique, des mono-ou disaccharides comme le glucose ou le saccharose, des polysaccharides, de l'isomalt, de la maltodextrine ou des polyols comme le maltitol.

Les comprimés à sucer sont des préparations unidoses solides destinées à être sucées afin d'exercer une action locale ou systémique. Ils sont obtenus par compression directe ou par granulation puis compression.
La composition pharmaceutique selon l'invention sera par exemple un comprimé à sucer, qui peut être préparé par un procédé de compression directe. Le comprimé à sucer selon l'invention contient de l'agomélatine, un agent diluant, et optionnellement un agent liant, un agent lubrifiant et/ou un agent d'écoulement et/ou un ou plusieurs agents édulcorants, aromatisants ou contre-irritants. Il est caractérisé par une érosion lente dans la cavité orale. Le diluant utilisable selon l'invention est choisi de telle sorte qu'il permette une utilisation en compression directe et qu'il confère à la forme pharmaceutique une forte résistance à l'écrasement et des propriétés organoleptiques acceptables pour une préparation buccale à sucer. Préférentiellement le diluant utilisé est un polyol ou un saccharide (mono-, oligo- ou polysaccharide) ou une combinaison de ces différents composés comme par exemple du glucose, du saccharose, du mannitol, des dextrates ou de la dextrine.
Parmi les agents liants utilisables selon l'invention, on citera les dérivés cellulosiques et les dérivés amylacés, la crospovidone, la maltodextrine.
Le contre-irritant utilisé optionnellement est un composé agissant sur les récepteurs vanilloïdes ou sur les récepteurs ASICs (acid-sensitizing ion channels) responsables des sensations d'irritation et de douleur. Le contre-irritant préférentiellement utilisé est l'acide citrique.
Les autres excipients utilisés sont les excipients classiquement décrits pour chacune des classes dans les ouvrages de référence comme par exemple le Handbook of Pharmaceutical Excipients (Rowe, Sheskey et Owen, Pharmaceutical Press).
Parmi les agents lubrifiants utilisables selon l'invention, on citera le stéarate de magnésium, l'acide stéarique, le béhénate de glycérol, les sucro-esters ou le stéaryl fumarate de sodium, et plus préférentiellement le stéarate de magnésium ou le stéaryl fumarate de sodium.
Parmi les édulcorants optionnellement envisagés selon l'invention, on citera l'aspartame, l'acésulfame potassium, le sucralose ou la saccharine.
Parmi les aromatisants selon l'invention optionnellement envisagés, on comprendra tout composé destiné à agir sur les descripteurs du goût (salé, sucré, amer, acide, umami).

Les comprimés sublinguaux et gingivaux sont des préparations unidoses solides respectivement destinées à être appliquées sous la langue et dans l'espace gingivo-jugal, en vue d'une action systémique. Ils sont fabriqués par compression de mélanges de poudres ou de granulés de façon à obtenir des comprimés de forme adaptée à l'usage qui en est prévu.
Les comprimés sublinguaux et gingivaux selon l'invention comprendront les mêmes ingrédients que les comprimés à sucer. Ils présentent des profiles de libération similaires. Ils ne diffèrent des comprimés à sucer que par la voie d'administration. Les comprimés à sucer sont destinés à une administration par voie oromucosale, c'est-à-dire que l'absorption du principe actif se fait via l'ensemble des muqueuses de la cavité buccale, tandis que les comprimés sublinguaux sont appliqués spécifiquement par voie sublinguale, c'est-à-dire sous la langue, et les comprimés gingivaux spécifiquement par voie gingivale, c'est-à-dire par les gencives.

Les solides obtenus par extrusion à chaud ou injection-moulage sont préparés avec les mêmes ingrédients que les comprimés à sucer. Cependant, des polymères thermoplastiques sont spécifiquement ajoutés afin de conférer au mélange d'ingrédients des propriétés d'écoulement à chaud. La mise en forme du solide est obtenue par extrusion à travers une matrice puis calendrage et refroidissement ou par injection de la matière dans un moule refroidi. Les polymères thermoplastiques utilisés selon l'invention sont les composés classiquement décrits dans les ouvrages de référence comme par exemple le Polymer Handbook (Brandrup, Immergut et Grulke, Wiley-Interscience). On citera par exemple les dérivés cellulosiques, les polyoxyéthylènes, les polyvinylpyrrolidones ou les poly métacrylates. Optionnellement, des composés plastifiants peuvent être utilisés afin de faliciter l'écoulement et la mise en forme du matériau. Les plastifiants utilisés selon l'invention sont les plastifiants classiquement décrits dans les ouvrages de référence comme par exemple le Handbook of Pharmaceutical Excipients (Rowe, Sheskey et Owen, Pharmaceutical Press). On citera par exemple le dibutylsébaccate, la triacétine, le glycérol, le sorbitol ou les polyéthylène glycols.

La préparation buccale solide destinée à une action systémique selon l'invention, après administration, a montré une excellente acceptabilité du principe actif par le patient. En effet, la formulation mise au point selon l'invention permet une dissolution progressive de l'agomélatine, donc une libération lente et contrôlée évitant un apport massif particulièrement irritant de la substance active.
La demanderesse a de plus effectué des études pharmacocinétiques chez l'homme et a pu mettre en évidence de manière totalement inattendue et surprenante une bien meilleure biodisponibilité pour une composition pharmaceutique solide à administration buccale selon l'invention comparée aux comprimés orodispersibles à absorption oromucosale décrits dans l'art antérieur, pour des formulations de même dosage en principe actif et contenant le principe actif sous une même forme.

La composition pharmaceutique selon l'invention a donc permis d'augmenter très significativement la biodisponibilité du principe actif, de réduire par voie de conséquence la variabilité intra et inter-individuelle, tout en proposant au patient une formulation très bien acceptée.
L'invention concerne donc une composition pharmaceutique buccale solide contenant de l'agomélatine, destinée à une action systémique résultant en une augmentation de la biodisponibilité du principe actif comparé notamment à une forme orodispersible administrée en voie sublinguale ou oromucosale. Plus particulièrement, la composition pharmaceutique selon l'invention permet d'atteindre une biodisponibilité absolue du principe actif supérieure à 25%, et préférentiellement supérieure à 30%, correspondant à un gain d'un facteur proche de 1,5 à 2 par rapport aux formulationss orodispersibles sublinguales ou oromucosales de l'art antérieur, lorsque l'on compare des formulations de même dosage en principe actif et où le principe actif est sous la même forme.

Les compositions pharmaceutiques selon l'invention sont préférentiellement caractérisées en ce qu'elles contiennent, par rapport au poids total de la composition de 0,01% à 20% en poids d'agomélatine, plus particulièrement de 0,01% à 10%, et encore plus préférentiellement de 0,01% à 5%.

Les compositions pharmaceutiques selon l'invention contiennent préférentiellement un agent diluant présentant une très bonne aptitude à la compression tout en conférant un caractère érodable à la formulation. Parmi les agents diluants selon l'invention on citera les saccharides et les polyols, et plus préférentiellement le saccharose.

La posologie utile est adaptable selon la nature et la sévérité de l'affection, la voie d'administration ainsi que l'âge et le poids du patient. Cette posologie varie de 0,1 mg à 25 mg d'agomélatine par jour en une ou plusieurs prises, et préférentiellement de 0,1 mg à 10 mg d'agomélatine par jour en une ou plusieurs prises, et encore plus préférentiellement de 0,1 à 5 mg d'agomélatine par jour en une ou plusieurs prises.

Les exemples suivants illustrent l'invention mais ne la limitent en aucune façon :

### EXEMPLE 1

### Formulation : Comprimé à sucer terminé à 500 mg

| ***Constituants*** | ***Quantité* (*mg*)** |
|---|---|
| Agomélatine | 2 |
| Saccharose pour compression directe | 434,25 |
| Amidon de maïs prégélatinisé | 50 |
| Aspartam | 5 |
| Acesulfam potassium | 5 |
| Stéarate de magnésium | 3,75 |

Le comprimé à sucer est préparé par mélange des constituants (avec quatre étapes de prémélanges), suivi d'une compression directe avec une force de compression de 30 kN et avec une cadence de 30000 comprimés par heure. Les comprimés obtenus ont une dureté de 50 N.

Les exemples 2 et 3 sont obtenus selon le même procédé :

### EXEMPLE 2

### Formulation : Comprimé à sucer terminé à 500 mg

| ***Constituants*** | ***Quantité (mg)*** |
|---|---|
| Agomélatine | 0,5 |
| Saccharose pour compression directe | 435,75 |
| Amidon de maïs prégélatinisé | 50 |
| Aspartam | 5 |
| Acesulfam potassium | 5 |
| Stéarate de magnésium | 3,75 |

### EXEMPLE 3

### Formulation : Comprimé à sucer terminé à 500 mg

| ***Constituants*** | ***Quantité (mg)*** |
|---|---|
| Agomélatine | 1 |
| Saccharose pour compression directe | 435,25 |
| Amidon de maïs prégélatinisé | 50 |
| Aspartam | 5 |
| Acesulfam potassium | 5 |
| Stéarate de magnésium | 3,75 |

### EXEMPLE 4

### Formulation : Solide obtenu par injection-moulage

| ***Constituants*** | ***Quantité (mg)*** |
|---|---|
| Agomélatine | 2 |
| Hydroxypropyl cellulose | 333 |
| Mannitol | 150 |
| Aspartam | 7,5 |
| Acesulfam potassium | 7,5 |

Le solide est préparé par mélange des constituants. Le mélange est ensuite versé dans la trémie d'alimentation d'une presse pour injection-moulage de type Arburg S250. Le mélange est chauffé jusqu'à 140°C par les cinq zones de chauffé de l'unité de plastification réglées de façon croissante de 95°C à 140°C et injecté à 1500 bars dans un moule multi-empreinte.

### EXEMPLE 5

Une étude de pharmacocinétique en prise unique chez 12 volontaires sains de sexe masculin a été réalisée. Trois formulations, deux comprimés orodispersibles et un comprimé à sucer, ont été testées. Les formulations pharmaceutiques orodispersibles pouvant être administrées en oromucosale (sur la langue) ou sublinguale, les deux modes d'administration ont également été envisagés et évalués. Ainsi, les 12 volontaires ont reçu alternativement:
- un comprimé orodispersible à noyau central contenant 2 mg d'agomélatine administré en application sublinguale (noyau central contenant 2 mg d'agomélatine, 0,35 mg de stéarate de magnésium, 67,65 mg de Starlac® ; couche externe contenant 236,25 mg de Starlac®, 2,5 mg d'acesulfame potassium, 2,5 mg d'aspartame, 7,5 mg d'acide citrique et 1,25 mg de stéarate de magnésium);
- un comprimé orodispersible tricouche contenant 2 mg d'agomélatine administré en application sublinguale (noyau central contenant 2 mg d'agomélatine, 0,35 mg de stéarate de magnésium, 67,65 mg de Starlac® ; 2 couches externes de 125 mg chacune contenant 238,75 mg de Starlac®, 2,5 mg de sucralose, 7,5 mg d'acide citrique et 1,25 mg de stéarate de magnésium);
- un comprimé orodispersible tricouche contenant 2 mg d'agomélatine administré en application oromucosale;
- un comprimé à sucer selon l'invention contenant 2 mg d'agomélatine administré dans la cavité buccale (comprimé de l'Exemple 1).

Des prélèvements plasmatiques ont été effectués juste avant le traitement puis régulièrement de 2 minutes à 24 heures après l'administration du comprimé et les paramètres pharmacocinétiques suivants ont été mesurés : Cₘₐₓ (concentration maximale observée après administration) et AUC (bilan absorption-élimination du principe actif administré). Les résultats suivants ont été obtenus:

| | **Formulation** | **C**ₘₐₓ (ng/ml) | **AUC** (ng.h/ml) | Biodisponibilité absolue (%) |
|---|---|---|---|---|
| Art antérieur | Orodispersible à noyau central à 2 mg d'agomélatine Administration sublinguale | 8,3 ± 4,6 (7,8) | 5,7 ± 2,7 (5,4) | 19% ±8,6 |
| | Orodispersible tricouche à 2 mg d'agomélatine Administration sublinguale | 7,8 ± 3,4 (7,5) | 5,1±2,1 (5,2) | 17% ± 6,6 |
| | Orodispersible tricouche à 2 mg d'agomélatine Administration oromucosale | 8,7 ± 2,5 (8,7) | 6,1 ± 1,6 (6,1) | 20% ± 4,9 |
| Exemple 1 | Comprimé à sucer à 2 mg d'agomélatine Administration buccale | 16 ± 4,9 (16,0) | 11 ± 2,8 (11,0) | 38% ± 8,9 |

Les résultats obtenus montrent que la formulation mise au point selon la présente invention permet de doubler les paramètres pharmacocinétiques obtenus avec les formulations orodispersibles décrites précédemment. L'AUC, directement corrélée à la biodisponibilité, montre que cette nouvelle formulation permet de doubler la biodisponibilité obtenue par rapport au comprimé orodispersible.

### EXEMPLE 6

Une seconde étude de pharmacocinétique en prise unique chez 12 volontaires sains de sexe masculin a été réalisée. Quatre formulations, deux comprimés orodispersibles et deux comprimés à sucer, ont été testées. Les formulations pharmaceutiques orodispersibles ont été administrées en sublinguale. Ainsi, les 12 volontaires ont reçu alternativement:
- un comprimé à sucer contenant 0,5 mg d'agomélatine administré dans la cavité buccale (comprimé de l'Exemple 2);
- un comprimé à sucer contenant 1 mg d'agomélatine administré dans la cavité buccale (comprimé de l'Exemple 3);
- un comprimé orodispersible à noyau central contenant 0,5 mg d'agomélatine administré en sublingual (noyau central contenant 0,5 mg d'agomélatine, 0,35 mg de stéarate de magnésium, 69,15 mg de Starlac® ; couche externe contenant 236,25 mg de Starlac®, 2,5 mg d'acesulfame potassium, 2,5 mg d'aspartame, 7,5 mg d'acide citrique et 1,25 mg de stéarate de magnésium);
- un comprimé orodispersible à noyau central contenant 1 mg d'agomélatine administré en sublingual (noyau central contenant 1 mg d'agomélatine, 0,35 mg de stéarate de magnésium, 68,65 mg de Starlac® ; couche externe contenant 236,25 mg de Starlac®, 2,5 mg d'acesulfame potassium, 2,5 mg d'aspartame, 7,5 mg d'acide citrique et 1,25 mg de stéarate de magnésium).

Des prélèvements plasmatiques ont été effectués juste avant le traitement puis régulièrement de 2 minutes à 24 heures après l'administration du comprimé et les paramètres pharmacocinétiques suivants ont été mesurés : Cₘₐₓ (concentration maximale observée après administration) et AUC (bilan absorption-élimination du principe actif administré). La biodisponibilité relative (AUC_{comprimé selon l'invention} / AUC_{comprimé orodispersible de l'art antérieur}) a été calculée pour chaque individu et les résultats obtenus ont été moyennés. L'ensemble des résultats obtenus est reporté dans le tableau suivant:

| | **Formulation** | **C**ₘₐₓ (ng/ml) | **AUC** (ng.h/ml) | Biodisponibilité relative (F_{comprimé à sucer} / F_{comprimé orodispersible}) |
|---|---|---|---|---|
| Art antérieur | Orodispersible à noyau central à 0,5 mg d'agomélatine Administration sublinguale | 3,6 ± 1,5 (3,2) | 2,7 ± 0,97 (2,6) | - |
| Exemple 2 | Comprimé à sucer à 0,5 mg d'agomélatine Administration buccale | 5,3 ± 1,2 (5,1) | 3,8 ± 0,93 (4,0) | 1,55 ± 0,65 (1,38) |
| Art antérieur | Orodispersible à noyau central à 1 mg d'agomélatine Administration sublinguale | 6,1 ± 2,6 (5,9) | 4,3 ± 1,9 (3,7) | - |
| Exemple 3 | Comprimé à sucer à 1 mg d'agomélatine Administration buccale | 9,4 ± 3,0 (9,0) | 7,3 ± 2,2 (7,0) | 1,86 ± 0,75 (1,54) |

Les résultats obtenus montrent que la formulation mise au point selon la présente invention, à différents dosages (0,5 et 1 mg de principe actif), permet d'augmenter significativement les paramètres pharmacocinétiques comparé à ceux obtenus avec les formulations orodispersibles décrites dans l'art antérieur aux mêmes dosages, et dans lesquelles le principe actif est sous la même forme. Ainsi, la biodisponibilité relative de la nouvelle formulation selon l'invention comparée à la formulation orodispersible de l'art antérieur montre une amélioration d'un facteur allant de 1,5 à 1,8.

## Revendications

1. Composition pharmaceutique buccale solide destinée à une action systémique contenant de l'agomélatine ou un de ses hydrates, complexes, co-cristaux, formes cristallines, sels d'addition à un acide ou une base pharmaceutiquement acceptable, et un ou plusieurs excipients choisis parmi des diluants, des liants, des agents d'écoulement, des lubrifiants, des agents d'enrobages, des plastifiants, des aromatisants, des contre-irritants et des édulcorants, **caractérisée en ce qu'**elle permet d'obtenir une libération lente du principe actif et une biodisponibilité absolue supérieure à 25%.

2. Composition pharmaceutique buccale solide destinée à une action systémique contenant de l'agomélatine ou un de ses hydrates, complexes, co-cristaux, formes cristallines, sels d'addition à un acide ou une base pharmaceutiquement acceptable, et un ou plusieurs excipients choisis parmi des diluants, des liants, des agents d'écoulement, des lubrifiants, des agents d'enrobages, des plastifiants, des aromatisants, des contre-irritants et des édulcorants, **caractérisée en ce qu'**elle permet d'obtenir une libération lente du principe actif et une biodisponibilité supérieure à celle obtenue pour un comprimé orodispersible à aborption oromucosale contenant le principe actif agomélatine sous la même forme et au même dosage.

3. Composition pharmaceutique selon la revendication 1 ou 2 **caractérisée en ce qu'**elle contient un agent diluant ayant une très bonne aptitude à la compression et conférant un caractère érodable.

4. Composition pharmaceutique selon la revendication 3 **caractérisée en ce que** l'agent diluant est un polyol ou un saccharide.

5. Composition pharmaceutique buccale solide destinée à une action systémique contenant de l'agomélatine ou un de ses hydrates, complexes, co-cristaux, formes cristallines, sels d'addition à un acide ou une base pharmaceutiquement acceptable, et un ou plusieurs excipients choisis parmi des diluants, des liants, des agents d'écoulement, des lubrifiants, des agents d'enrobages, des plastifiants, des aromatisants, des contre-irritants et des édulcorants, **caractérisée en ce qu'**elle est un comprimé à sucer.

6. Composition pharmaceutique selon l'une des revendications 1 à 4 qui est un comprimé à sucer.

7. Composition pharmaceutique buccale solide destinée à une action systémique contenant de l'agomélatine ou un de ses hydrates, complexes, co-cristaux, formes cristallines, sels d'addition à un acide ou une base pharmaceutiquement acceptable, et un ou plusieurs excipients choisis parmi des diluants, des liants, des agents d'écoulement, des lubrifiants, des agents d'enrobages, des plastifiants, des aromatisants, des contre-irritants et des édulcorants, **caractérisée en ce qu'**elle est une pastille à sucer.

8. Composition pharmaceutique selon l'une des revendications 1 à 4 qui est une pastille à sucer.

9. Composition pharmaceutique buccale solide destinée à une action systémique contenant de l'agomélatine ou un de ses hydrates, complexes, co-cristaux, formes cristallines, sels d'addition à un acide ou une base pharmaceutiquement acceptable, et un ou plusieurs excipients choisis parmi des diluants, des liants, des agents d'écoulement, des lubrifiants, des agents d'enrobages, des plastifiants, des aromatisants, des contre-irritants et des édulcorants, **caractérisée en ce qu'**elle est un solide obtenu par extrusion à chaud ou injection-moulage.

10. Composition pharmaceutique selon l'une des revendications 1 à 4 qui est un solide obtenu par extrusion à chaud ou injection-moulage.

11. Composition pharmaceutique buccale solide destinée à une action systémique contenant de l'agomélatine ou un de ses hydrates, complexes, co-cristaux, formes cristallines, sels d'addition à un acide ou une base pharmaceutiquement acceptable, et un ou plusieurs excipients choisis parmi des diluants, des liants, des agents d'écoulement, des lubrifiants, des agents d'enrobages, des plastifiants, des aromatisants, des contre-irritants et des édulcorants, **caractérisée en ce qu'**elle est une pâte à sucer.

12. Composition pharmaceutique selon l'une des revendications 1 à 4 qui est une pâte à sucer.

13. Composition pharmaceutique selon l'une des revendications 1 à 12 **caractérisée en ce que** le principe actif utilisé pour sa préparation est obtenu sous la forme cristalline II.

14. Composition pharmaceutique selon l'une des revendications 1 à 13 **caractérisée en ce qu'**elle comprend de 0,1 à 25 mg d'agomélatine.

15. Composition pharmaceutique selon l'une des revendications 1 à 13 **caractérisée en ce qu'**elle comprend de 0,1 à 10 mg d'agomélatine.

16. Composition pharmaceutique selon l'une des revendications 1 à 13 **caractérisée en ce qu'**elle comprend de 0,1 à 5 mg d'agomélatine.

17. Composition pharmaceutique selon l'une des revendications 1 à 13 **caractérisée en ce qu'**elle comprend de 0,1 à 3 mg d'agomélatine.

18. Composition pharmaceutique selon l'une quelconque des revendications 1 à 17 **caractérisée en ce qu'**elle permet d'obtenir une biodisponibilité absolue supérieure à 30%.

19. Composition pharmaceutique selon l'une quelconque des revendications 1 à 17 **caractérisée en ce que** la biodisponibilité relative du comprimé à sucer par rapport à un comprimé orodispersible à obsorption oromucosale de même dosage et contenant le principe actif agomélatine exactement sous la même forme est d'au moins 1,5.

20. Composition pharmaceutique selon l'une quelconque des revendications 1 à 19, utile pour le traitement de la dépression majeure, des dépressions saisonnières, du trouble anxiété généralisée, du trouble obsessionnel compulsif, des troubles bipolaires, des troubles du sommeil, des pathologies cardiovasculaires, des pathologies du système digestif, des insomnies et fatigues dues aux décalages horaires, des troubles de l'appétit et de l'obésité et de l'ensemble des pathologies liées à un dérèglement des rythmes circadiens.
